# EUROPÄISCHE PATENTSCHRIFT

(11) **EP 2 761 018 B1**
(45) Veröffentlichungstag und Bekanntmachung des Hinweises auf die Patenterteilung: **08.03.2017**
(21) Anmeldenummer: 12761750.4
(22) Anmeldetag: 25.09.2012
(51) Int. Cl.: C12Q 1/68, B01L 7/00

(54) **SEQUENZSPEZIFISCHE ANALYSE VON NUKLEINSÄUREN**
SEQUENCE-SPECIFIC ANALYSIS OF NUCLEIC ACIDS
ANALYSE D'ACIDES NUCLÉIQUES SPÉCIFIQUE À UNE SÉQUENCE

(30) Priorität: 28.09.2011 DE 102011114984
(43) Veröffentlichungstag der Anmeldung: 06.08.2014
(73) Patentinhaber: Universität Rostock, 18055 Rostock (DE)
(72) Erfinder: FLECHSIG, Gerd-Uwe, 17166 Teterow (DE); MIX, Maren, 18057 Rostock (DE); KRÜGER, Simone, 18059 Rostock (DE)
(74) Vertreter: Moré, Solveig Helga
(86) Internationale Anmeldenummer: PCT/EP2012/068818
(87) Internationale Veröffentlichungsnummer: WO 2013/045417

(56) Entgegenhaltungen:
- WO-A1-2005/098438
- WO-A2-2007/020093
- US-A1- 2003 057 199
- US-A1- 2007 099 211
- US-A1- 2011 129 910
- NORDSTROM T ET AL: "Method enabling pyrosequencing on double-stranded DNA", ANALYTICAL BIOCHEMISTRY, ACADEMIC PRESS INC, NEW YORK, Bd. 282, Nr. 2, 1. Juli 2000 (2000-07-01), Seiten 186-193, XP008096692, ISSN: 0003-2697, DOI: 10.1006/ABIO.2000.4603
- Thomas Reske: "Voltammetrische Detektierung der DNA-Hybridisierung von Oligonukleotiden und PCR-Produkten an Goldelektroden mit Osmiumtetroxid als kovalentem Marker", , 1. Januar 2009 (2009-01-01), Seiten 1-84, XP055045629, Gefunden im Internet: URL:http://rosdok.uni-rostock.de/file/rosd ok_derivate_000000003880/Dissertation_Resk e_2009.pdf [gefunden am 2012-11-26] in der Anmeldung erwähnt
- DUWENSEE H ET AL: "Electrochemical product detection of an asymmetric convective polymerase chain reaction", BIOSENSORS AND BIOELECTRONICS, ELSEVIER BV, NL, Bd. 25, Nr. 2, 15. Oktober 2009 (2009-10-15), Seiten 400-405, XP026600441, ISSN: 0956-5663, DOI: 10.1016/J.BIOS.2009.07.025 [gefunden am 2009-08-03]
- RESKE ET AL: "Electrochemical detection of osmium tetroxide-labeled PCR-products by means of protective strands", TALANTA, ELSEVIER, AMSTERDAM, NL, Bd. 74, Nr. 3, 11. Dezember 2007 (2007-12-11), Seiten 393-397, XP022384832, ISSN: 0039-9140, DOI: 10.1016/J.TALANTA.2007.09.004 in der Anmeldung erwähnt
- FLECHSIG GERD-UWE ET AL: "Electrochemical detection of DNA hybridization by means of osmium tetroxide complexes and protective oligonucleotides", ANALYTICAL CHEMISTRY, AMERICAN CHEMICAL SOCIETY, US, Bd. 79, Nr. 5, 1. März 2007 (2007-03-01), Seiten 2125-2130, XP002425409, ISSN: 0003-2700, DOI: 10.1021/AC062075C in der Anmeldung erwähnt
- PALECEK E ET AL: "DETECTING DNA HYBRIDIZATION AND DAMAGE", ANALYTICAL CHEMISTRY, AMERICAN CHEMICAL SOCIETY, US, Bd. 73, Nr. 3, 1. Februar 2001 (2001-02-01), Seiten 74A-83A, XP009080149, ISSN: 0003-2700

## Beschreibung

Die Erfindung betrifft ein Verfahren zur sequenzspezifischen Analyse von Nukleinsäuren in einer Probe, in der die Nukleinsäure zumindest partiell als Doppelstrang vorliegt, welches elektrochemische Detektionsverfahren (z.B. mit [OsO₄(bipy)]) nutzt. Insbesondere umfassen die Verfahren Schritte, bei denen man die zumindest partiell doppelsträngigen Nukleinsäurestränge durch thermische Denaturierung in Einzelstränge überführt, welche als Targetstränge bezeichnet werden, und mindestens einen als Schutzstrang bezeichneten Nukleinsäurestrang hinzufügt, welcher mit einem Targetstrang hybridisieren kann, um partielle Doppelstrangabschnitte auszubilden, wobei die Schutzstränge kürzer als die Targetstränge sind, wobei die Temperatur der Probe schnell auf eine Temperatur weniger als 0 °C gesenkt wird. Die Offenbarung betrifft auch Vorrichtungen, die für diese Verfahren geeignet sind, umfassend ein Fließsystem mit heizbaren Abschnitten zur thermischen Denaturierung sowie kühlbaren Abschnitten zur schnellen Kühlung der Probe, in welchen die Schritte des Verfahrens nacheinander stattfinden können.

### Stand der Technik

Um eine elektrochemische Detektierung von Hybridisierungsereignissen von Nukleinsäuren zu ermöglichen, können redoxaktive Moleküle eingesetzt werden, die entweder an den Target- oder Reporterstrang kovalent gebunden sind, sich elektrostatisch an die Phosphatgruppen der Nukleinsäuren binden oder sich als Interkalatoren in den Doppelstrang einlagern. Die kovalent gebundenen so genannten Redoxmarker werden bei der Markierung oder Herstellung der Target- oder Reporterstränge angebunden. Alternativ kann Osmium(VIII)oxid in Form von Komplexen mit Bipyridin eingesetzt werden. Diese Komplexverbindungen reagieren spezifisch mit den Pyrimidinbasen Thymin, Uracil und in weitaus geringerem Maße mit Cytosin. Diese Reaktion findet in einem intakten Doppelstrang nicht statt. Andererseits können Einzelstränge, deren Pyrimidinbasen mit Osmiumkomplexen reagiert haben, keine Doppelstränge mehr bilden. Diese Zusammenhänge wurden seit Anfang der 80er Jahre von Palecek, Jelen und Fojta untersucht und in Form zahlreicher Beispiele zur Detektion der Nukleinsäuren verwendet (M. Foita et al. J. Am. Chem. Soc. 126 (2004) 6532; P. Kostecka et al. Bioelectrochemistry 63 (2004) 245; M. Foita et al. Electroanalysis 15 (2003) 431).

Die Modifikation von DNA mittels Osmium(VIII)-Komplexen kann sehr einfach durch Zugeben des Osmiumreagenzes zur Analysenlösung erfolgen. Nach der Modifizierung wird das überschüssige Reagenz durch Dialyse in handelsüblichen einfachen Dialysegefäßen abgetrennt. Weiterhin wird üblicherweise die Hybridisierung von Target und Sonde an unterschiedlichen Oberflachen durchgeführt. Besonders geeignet sind so genannte Magnetic Beads, magnetische Kügelchen, welche an ihrer Oberfläche immobilisierte Sondenstränge tragen und mittels Magnetfeld von der Analyselösung (und damit von nicht komplementären Strängen und Analysereagenzien) getrennt werden können. Nach diesem Prinzip kann man beliebige einzelsträngige Nukleinsaurestränge markieren. Nukleinsäuren in der Mehrzahl der Proben liegen jedoch in doppelsträngiger Form vor. Dies gilt sowohl für DNA als auch für RNA; welche aufgrund von Sekundärstrukturen in großen Teilen doppelsträngig ist.

Es besteht das Problem, dass einerseits osmiummodifizierte Nukleinsäureeinzelstränge nicht mehr zur Doppelstrangbildung fähig sind, und dass andererseits intakte Doppelstränge nicht mit den Osmiumtetroxid-Komplexen wie dem [OsO₄(bipy)] reagieren. Es wurden im Stand der Technik verschiedene Wege gefunden, dieses Problem zu lösen.

Dies kann dadurch gelöst werden, dass ein Schutzstrang den Teil des Einzelstranges blockiert, der später mit einer Sonde hybridisieren soll. Auf diese Weise gelingt die Markierung von PCR-Produkten oder nativen Nukleinsäureproben mit Osmium, ohne dass die Fähigkeit zur Hybridisierung verloren geht. Die große Zahl der an die Targetstränge gebundenen Osmiumeinheiten bewirkt eine sehr hohe Empfindlichkeit bei der anschließenden elektrochemischen Analyse (WO 07/020093). Dort wurden z.B. mit einer PCR amplifizierte Doppelstränge thermisch denaturiert (95 °C), der Schutzstrang im Überschuss zugesetzt und auf 35 °C abgekühlt. Es wird gelehrt, dass die optimale Hybridisierungstemperatur unterhalb der Schmelztemperatur liegt und mit einer Schmelzkurvenanalyse festgestellt werden kann.

Der erste große Vorteil einer Markierung von Nukleinsäuren mit Hilfe von Osmiumtetroxidkomplexen besteht in der einfachen Durchführung. Man muss lediglich die DNA/RNA-haltige Lösung mit dem Komplex, z.B. [OsO₄(bipy)] versetzen, dann reagiert diese Verbindung spontan mit allen Thymin-Basen in der Lösung, die nicht in DNA-Doppelsträngen vorliegen. Auch andere Pyrimidinbasen werden auf diese Weise angegriffen. Cytosin reagiert allerdings etwa 10-mal langsamer als Thymin. In der RNA tritt die Pyrimidinbase Uracil an die Stelle des Thymins. Sie unterscheidet sich vom Thymin lediglich durch eine fehlende Methylgruppe und reagiert etwas langsamer mit Osmiumtetroxid-Bipyridin.

Der zweite große Vorteil ist die hohe Empfindlichkeit, die erreicht wird, wenn der Targetstrang viele Thymin- bzw. Uracilbasen enthält. Denn der größte Teil hiervon wird stets im Einzelstrangabschnitt auftreten und damit vom [OsO₄(bipy)] angegriffen werden. Im Schnitt kann man bei einem PCR-Produkt mit 50 bis 200 Thyminbasen pro Strang rechnen. RNA kann über 500 Uracilbasen enthalten.

Flechsig et al., Anal. Chem. 2007, zeigen, dass eine Hybridisierung von Einzelsträngen mit Schutzsträngen optimal bei 40 °C abläuft. Reske et al., Talanta 2007, analysierten doppelsträngige PCR-Produkte nach Generierung von Einzelsträngen durch Lambda-Exonuklease-Behandlung, vor Hybridisierung der Einzelstränge mit kurzen Schutzsträngen bei Raumtemperatur (RT) für 2 h und anschließender Reaktion mit [OsO₄(bipy)] für 2 h bei RT.

Mix et al., Electroanalysis 2009, beschreiben die Herstellung einzelsträngiger DNA-Stränge durch asymmetrische PCR, gefolgt wiederum von Hybridisierung der Einzelstränge mit kurzen Schutzsträngen bei Raumtemperatur (RT) für 2 h und anschließender Reaktion mit [OsO₄(bipy)] für 2 h bei RT.

**Aufgabe der Erfindung** ist es daher, ein Verfahren zu schaffen, welche eine schnelle und einfache Detektion der gesuchten Nukleinsäuresequenzen mit Redoxmarkern wie Osmiumtetroxid-Komplexen gegebenenfalls auch ohne vorherige PCR ermöglichen, sowie direkt anschließend die elektrochemische Detektierung an einer Arbeitselektrode gestatten, wobei auf eine asymmetrische PCR, Exonuklease-Verdau oder ähnliche Verfahren zur Generierung von Einzelsträngen verzichtet werden kann. Dies ist insbesondere dann von Vorteil, wenn die gesuchten Sequenzen in der Probe zumindest partiell als Doppelstränge vorliegen, z.B. in Gegenwart eines großen Überschusses an unspezifischer DNA bzw. RNA. Doppelsträngige DNA lässt sich vor der Detektion oft vorteilhaft durch eine klassische, symmetrische PCR vervielfältigen, welche eine bessere Ausbeute ergibt als asymmetrische PCR, und damit zu einer verbesserten Selektivität und Empfindlichkeit führt.

### Lösung der Aufgabe

Gelöst wird diese Aufgabe durch den Gegenstand der Ansprüche 1 bis 12, insbesondere durch ein Verfahren zum sequenzspezifischen Nachweis von Nukleinsäure in einer Probe, in der die Nukleinsäure zumindest partiell als Doppelstrang vorliegt, welches Schritte umfasst, bei denen man
a) die zumindest partiell doppelsträngigen Nukleinsäurestränge durch thermische Denaturierung in Einzelstränge überführt, welche als Targetstränge bezeichnet werden, wobei die thermische Denaturierung bei mindestens 93°C für mindestens 10 min stattfindet;
b) mindestens einen als Schutzstrang bezeichneten Nukleinsäurestrang hinzufügt, welcher mit einem Targetstrang hybridisieren kann, um partielle Doppelstrangabschnitte auszubilden, wobei die Schutzstränge kürzer als die Targetstränge sind, wobei die Temperatur der Probe schnell auf eine Temperatur von weniger als 0°C gesenkt wird, indem die Probe sofort nach Hinzufügen des Schutzstrangs in Schritt b) in eine Umgebung von weniger als 0°C überführt wird, wobei die Temperatur mindestens 10 min gehalten wird,
c) die verbliebenen Einzelstrangabschnitte der Targetstränge durch Reaktion mit einem Redoxmarker markiert, der selektiv mit der Doppelbindung der Pyrimidinringe der Nukleinsäurestränge reagieren und eine elektroanalytisch nutzbare Redoxreaktion an Arbeitselektroden ermöglicht, wobei der Redoxmarker [Os0₄ (bipy)] oder [Os0₄ (py)₂ ] ist, man
d) die so markierten Nukleinsäurestränge an der Oberfläche einer Elektrode mit dort immobilisierten Sondensträngen unter Verdrängung der Schutzstränge hybridisiert und man
e) die an den Sondensträngen hybridisierten Nukleinsäurestränge elektroanalytisch nachweist.

Das Grundprinzip der vorliegenden Erfindung besteht darin, sämtliche Nukleinsäuren (DNA und/oder RNA) einer Probe zu Einzelsträngen zu denaturieren und die unspezifischen (nicht gesuchten) Abschnitte mit Osmiumtetroxid-Komplexen zu blockieren, so dass sie nicht mehr zur Hybridisierung in der Lage sind. Die gesuchten Sequenzen werden dagegen auf an sich bekannte Weise mit kurzen Schutzsträngen geschützt (s.a. DE 10 2005 039 726 oder WO 07/020093).

Erfindungsgemäß wird dabei die unterschiedliche Hybridisierungsgeschwindigkeit verschieden langer Nukleinsäurestränge ausgenutzt. So bleiben die langen DNA und RNA-Stränge nach beispielsweise thermischer Denaturierung und Abschrecken im Eisbad oder Kältemischung für längere Zeit als Einzelstränge erhalten, während die kurzen Schutzstränge (typischerweise 20 bis 30 Basen) sehr viel schneller hybridisieren können. Durch thermisches Aufschmelzen der Doppelstrang-DNA und anschließendes schnelles Abkühlen können die langen Einzelstränge längere Zeit wegen der kinetischen Hemmung nicht rehybridisieren. Ein Vergleich mit Reske, Dissertation, 2009, zeigt, dass erst ein sehr schnelles Abkühlen auf niedrige Temperatur, verbunden mit entsprechendem ausreichend langen Halten der hohen bzw. niedrigen Temperatur zum Erfolg führt.

Im Rahmen der Erfindung werden in dieser Phase Schutzstränge hinzugefügt, die kurze Doppelstrangabschnitte bilden können. Die so geschützten Abschnitte werden bei der darauf folgenden Modifizierung mit Osmiumtetroxid-Komplexen nicht angegriffen und stehen später für die Hybridisierung mit Fängersonden zur Verfügung. Alle übrigen DNA- oder RNA-Abschnitte liegen nach wie vor als Einzelstränge vor und werden nun mittels Osmiumtetroxid-Komplexen modifiziert. Diese Einzelstrangabschnitte sind nun nicht mehr zur Hybridisierung fähig.

Auf diese Weise lassen sich erfindungsgemäß alle ungeschützten DNA- oder RNA-Abschnitte ausblenden, wenn es anschließend um die Hybridisierung mit Fängersonden an Goldelektroden geht. Es wird die unspezifische Anlagerung von nicht komplementären Nukleinsäuresträngen an die Sondenstränge erschwert. Die Erkennung der Targetstränge in einem Überschuss an nichtkomplementären Nukleinsäuresträngen wird daher erleichtert. Erfindungsgemäß erfolgt zweimal eine Selektion der gesuchten Nukleinsäuresequenz: Bei der Hybridisierung mit den Schutzsträngen sowie bei der Hybridisierung mit den immobilisierten Fängersonden.

Bevorzugt findet in dem erfindungsgemäßen Verfahren die thermische Denaturierung in Schritt a) bei 95 °C oder 97 °C für mindestens 10 min statt. Besonders bevorzugt findet eine thermische Denaturierung bei 95 °C für mindestens 10 min statt.

Das Hinzufügen des Schutzstranges in Schritt b) erfolgt erfindungsgemäß etwa bei der Temperatur der thermischen Denaturierung. Insbesondere kann es genau bei dieser Temperatur erfolgen oder direkt nachdem die Probe einer Umgebung dieser Temperatur entnommen wurde. Die Temperatur der Probe beim Hinzufügen des Schutzstranges ist also bevorzugt etwa 80-95 °C, besonders bevorzugt etwa 90-95 °C.

Die Zeiten für Denaturierung der Doppelstrang-Abschnitte der DNA- oder RNA-Targets und für die Hybridisierung mit Schutzsträngen sind mindestens 10 min. Im Rahmen des erfindungsgemäßen Verfahrens wird die Temperatur der Probe anschließend schnell auf eine Temperatur von 0 °C, bevorzugt auf ca. -1 °C bis -5 °C, auf -2° C bis -4 °C, oder auf -2 °C bis -3 ° C, insbesondere auf etwa -2,5° C, gesenkt, indem die Probe sofort nach Hinzufügen des Schutzstrangs in Schritt b) in eine Umgebung von weniger als 0 °C überführt wird. Da die Temperatur der Probe auf weniger als 0 °C gesenkt wird, beträgt die Umgebungs-Temperatur in Schritt b) bevorzugt ca. -5 °C bis -25 °C, bevorzugt etwa -19 °C. Solche Temperaturen können z.B. in einer Kältemischung aus Eis und einem Salz erreicht werden, wobei das Salz bevorzugt NaCl ist und die Salzlösung gesättigt ist. Eine derartige Kältemischung kann z.B. eine Temperatur von -19 °C aufweisen. Die schnelle Temperatursenkung in Schritt b) wird also durch die geringe Umgebungstemperatur (z.B. in einer Kältemischung) und eine gute Wärmeübertragung (wie z.B. in der Kältemischung) erreicht.

Die Temperatur in Schritt b) wird mindestens 10 min gehalten. Danach ist eine weitere Hybridisierung der Schutzstränge bei höheren Temperaturen, insbesondere bei RT (ca. 20-25 °C) möglich. Dies kann weitere 5-120 min erfolgen, insbesondere 10-110 min oder 30-60 min. Unter "partiellen Doppelstrangabschnitte", die durch Schutzstrang und Targetstrang in Schritt b) gebildet werden, versteht man, dass die einzelsträngigen Targetstränge nur über einen Teil ihrer Gesamtlänge mit Schutzsträngen hybridisieren und sich dadurch doppelsträngige Abschnitte bilden, d.h., die Länge der Schutzstränge wird so gewählt, dass noch ausreichend lange einzelsträngige Abschnitte der Targetsequenz für die nachfolgende Reaktion mit den Redoxmarkern zur Verfügung stehen. Diese verbleibenden einzelsträngigen Abschnitte der Targetsequenz müssen mindestens eine Thyminbase enthalten. Je mehr Thyminbasen im Einzelstrangabschnitt vorliegen, desto mehr können im anschließenden Schritt mit dem Redoxmarker markiert werden und desto höher werden zum Schluss die elektroanalytischen Signale und damit die Empfindlichkeit des ganzen Verfahrens sein.

Die Hybridisierung erfolgt mit Schutzsträngen, die kürzer als die Targetstränge sind. Insbesondere ist der Schutzstrang höchstens halb so lang wie der Targetstrang, höchstens ¼ so lang oder höchstens 1/10 so lang. Der Schutzstrang weist bevorzugt eine Länge von 10-200 b auf, mehr bevorzugt von 15-100 b oder 20-30 b. Die Länge der Schutzstränge muss dabei ausreichend sein, um eine ausreichend feste Doppelstrangbildung zu ermöglichen. Andererseits erfolgt in Schritt d) eine Hybridisierung mit Sondensträngen, die an der Oberfläche einer Elektrode immobilisiert sind. Da diese Hybridisierung unter Verdrängung der Schutzstränge erfolgt, ist folgendes zu beachten: Die Sondenstränge sollten genauso lang sein wie die Schutzstränge oder nur geringfügig länger. Es ist von Vorteil, wenn im Target-Schutzstrang-Duplex Fehlpaarungen auftreten. Dieser Vorteil kommt insbesondere zum Tragen, wenn die Sondenstränge etwas kürzer als die Schutzstränge sind. Die Fehlpaarungen sollten nicht an Stellen des Targetstranges auftreten, an denen Thymin-Basen sitzen, da diese sonst markiert werden könnten, was eine spätere Hybridisierung mit den Sondensträngen beeinflussen würde. So wird gewährleistet, dass der Targetstrang-Schutzstrang-Duplex nicht stabiler als der Targetstrang-Sondenstrang-Duplex ist, und dass ein Tausch der Schutzstränge gegen die Sondenstränge stattfindet.

Bevorzugt wird in Schritt b) Schutzstrang in einem Überschuss von mindestens 2:1, bevorzugt 3:1, 4:1, 5:1, 9:1 oder 10:1 hinzugefügt.

Im Rahmen des erfmdungsgemäßen Verfahrens ist der Redoxmarker, der selektiv mit der Doppelbindung der Pyrimidinringe der Nukleinsäurestränge reagiert und eine elektroanalytisch nutzbare Redoxreaktion an Arbeitselektroden ermöglicht, ein Osmium(VIII)-Komplex, [OsO₄(bipy)] oder [OsO₄(py)₂], insbesondere [OsO₄(bipy)]. Alternative Redoxmarker sind in WO 07/020093 offenbart.

Die Nukleinsäure in der Probe kann RNA und/oder DNA sein. Sowohl DNA als auch RNA ist im Allgemeinen zumindest partiell doppelsträngig, insbesondere bei Raumtemperatur (ca. 20-25 °C). Bei RNA beruht dies insbesondere auf Sekundärstrukturen.

Die Nukleinsäure der Targetstränge kann eine Länge von 100 bis 5000 Basenpaaren, vorzugsweise 500 bis 2000 Basenpaaren aufweisen, oder länger sein. In einer Ausführungsform wird die Nukleinsäure in der Probe vor der Denaturierung in Schritt a) in kürzere Abschnitte geteilt, z.B. von jeweils 100 bis 5000 Basenpaaren, vorzugsweise 500 bis 2000 Basenpaaren. Dies kann z.B. mit Nuklease-Behandlung oder durch Ultraschall erreicht werden.

Bevorzugt erfolgt die Verdrängung der Schutzstränge durch die immobilisierten Sondenstränge in Schritt d) bei einer Temperatur, die optimal für die thermisch stringente Hybridisierung von Sonden- und Targetsträngen ist. Bevorzugt sind die Sondenstränge an einer zum Zeitpunkt der Messung und/oder Hybridisierung beheizten Elektrode (Arbeitselektrode) immobilisiert. Dadurch wird einerseits der Strangaustausch an der Elektrodenoberfläche beschleunigt und andererseits die Anbindung von nicht hundertprozentig komplementären Strängen an die Sondenstränge erschwert. Die optimale Temperatur liegt unterhalb der sogenannten Schmelztemperatur. Bei letzterer liegen die beiden komplementären Stränge zu 50% als Doppelstrang vor. Zur Ermittlung der optimalen Temperatur eines jeden Sondenstranges bedient man sich der Schmelzkurvenanalyse. Dabei wird ein Analysensignal, welches vom Hybridisierungszustand abhängt, gegen die Temperatur aufgetragen. In homogener Lösung werden dazu üblicherweise die UV-Absorption oder die Fluoreszenz der DNA gemessen. Falls gewünscht, können zum Nachweis mehrerer Sequenzen in einer Probe mehrere unterschiedliche Sonden, welche zu den nachzuweisenden Sequenzen komplementär sind, verwendet werden. Bevorzugt sind diese unterschiedlichen Sonden an unterschiedlichen selektiv beheizten Elektroden immobilisiert, um während der Hybridisierung und/oder Messung die für jede Sondensequenz jeweils optimale Temperatur einzustellen.

In einer Ausführungsform werden zwischen Schritt c) und d) die überschüssigen Redoxmarker aus der Analysenlösung entfernt, z.B. durch Dialyse oder Filtration.

Gemäß einer weiteren Verfahrensvariante verbleiben die überschüssigen Redoxmarker nach Schritt c) in der Analysenlösung, d.h. zwischen Schritt c) und d) werden die überschüssigen Redoxmarker aus der Analysenlösung nicht entfernt, und man trennt die elektrochemischen Signale der an der Oberfläche der Elektrode gebundenen Redoxmarker von den Signalen des in Lösung befindlichen Redoxmarkers bei der Detektierung durch geeignete elektrochemische Analyseverfahren voneinander. Bei dieser Verfahrensvariante ist die Chronocoulometrie oder Chronopotentiometrie als elektrochemisches Analyseverfahren bevorzugt. Die Chronocoulometrie gestattet die Unterscheidung zwischen elektrochemischen Diffusionsströmen, welche von der Umsetzung gelöster Substanzen stammen und elektrochemischen Strömen, die von oberflächlich fixierten Substanzen verursacht werden. Auf diese Weise lässt sich z.B. die Menge immobilisierter DNA auf Goldelektroden bestimmen, indem man Rutheniumhexamminchlorid zur Lösung dazu setzt. Dieses bindet sich an die Phosphatgruppen der DNA-Stränge (vgl. z.B. Steel, B.A. et al. Anal. Chem. 1998, 70, 4670-4677).

Gemäß einer Ausführungsform liegen die Sondenstränge in Schritt d) in hybridisierter Form vor, d.h. sie sind mit einem oder mehreren Nukleinsäuresträngen als Sondenschutzsträngen hybridisiert. In diesem Fall hybridisiert man in Schritt d) die markierten Targetnukleinsäurestränge an der Oberfläche einer Elektrode mit den dort immobilisierten Sondensträngen unter Verdrängung der Schutzstränge und der Sondenschutzstränge.

Der elektroanalytische Nachweis der Targetstränge, welche an die immobilisierten Sondenstränge angebunden sind, erfolgt im Rahmen des erfindungsgemäßen Verfahrens vorzugsweise mittels Chronopotentiometrie, Coulometrie, Amperometrie oder Voltammetrie, vorzugsweise mittels Square-Wave-Voltammetrie (SWV), Wechselstromvoltammetrie (ACV) oder cyclischer Voltammetrie (CV). Als Elektrode verwendet man Metall-, Kohle-, Polymer- oder Halbleiterelektroden, vorzugsweise Draht- oder Schichtelektroden, welche aus Gold, Platin, Kupfer, Bismut, Quecksilber, Silber, Blei, Zinn bzw. deren Legierungen bestehen. Bevorzugt kommen Elektroden zum Einsatz, welche mittels elektrischen Stromes direkt oder mittels eines Widerstandsheizers indirekt heizbar sind. Bevorzugt ist eine beheizbare Elektrode direkt beheizbar und weist eine gleichmäßige Oberflächentemperatur auf.

Die Elektrodenoberflächen können mit Strukturen im Nano- oder Mikrometerbereich modifiziert sein, um ihre Oberfläche zu vergrößern. Diese Strukturen können aus dem gleichen Material sein wie die Elektrode selbst. Die Immobilisierung der Sondenstränge auf der Oberfläche der Elektroden erfolgt nach dem Fachmann bekannten Verfahren. Dabei ist es erfindungsgemäß bevorzugt, die Sondenstränge auf Metallelektroden vorzugsweise mittels Thiolgruppen zu chemisorbieren (vgl. z.B. Steel, B.A.; Herne, T.M.; Tarlov, M.J. Anal. Chem. 1998, 70, 4670-4677).

Gegenstand der Offenbarung ist auch eine Vorrichtung zur Durchführung eines erfindungsgemäßen Verfahrens, umfassend ein Fließsystem, in welchem die genannten Schritte des Verfahrens nacheinander stattfinden können, wobei es in dem Fließsystem heizbare Abschnitte zur thermischen Denaturierung sowie kühlbare Abschnitte zur schnellen Kühlung der Probe gibt. Insbesondere ist der Abschnitt zur thermischen Denaturierung auf 93-97 °C, bevorzugt 95 °C beheizbar oder beheizt. In dem Fließsystem wird der Probe direkt danach Schutzstrang hinzugefügt, d.h., das System ist so ausgestaltet, dass Schutzstrang hinzugefügt werden kann, z.B. durch eine Kanalmündung oder Einspritzvorrichtung. Auch dieser Bereich kann noch auf die Temperatur zur thermischen Denaturierung oder eine leicht darunter liegende Temperatur beheizbar oder beheizt sein. Das Fließsystem ist so ausgestaltet, dass die Probe direkt danach in einen Abschnitt geleitet werden kann, der auf eine Temperatur gekühlt ist, die dazu geeignet ist, die Temperatur der Probe auf von 0 °C oder weniger, bevorzugt auf etwa -1 °C bis etwa -5 °C, -2 °C bis -4 °C oder etwa -2,5 °C zu senken. Der Abschnitt kann dafür z.B. auf -2,5 °C bis - 25 °C oder etwa -5° C bis -19 °C kühlbar sein oder gekühlt sein. Der Gefrierpunkt der Probe sollte in der Probe nicht unterschritten werden, während sie diesen gekühlten Abschnitt passiert. Durch die schnelle Abkühlung der Probe kommen die bereits erläuterten vorteilhaften Effekte bei der Anlagerung der Schutzstränge zum Tragen.

In einer Ausführung umfasst die Vorrichtung eine Probe, welche Nukleinsäuren umfasst, und die oben erwähnten heizbaren und kühlbaren Abschnitte sind auf die entsprechenden Temperaturen geheizt bzw. gekühlt.

In der Vorrichtung wird bevorzugt als elektrochemischer Detektor zum elektroanalytischen Nachweis der an den Sondensträngen hybridisierten Nukleinsäurestränge ein Array aus unterschiedlichen selektiv heizbaren Arbeitselektroden verwendet, wobei an den Arbeitselektroden unterschiedliche Sonden (d.h. Sonden mit unterschiedlicher Sequenz) immobilisiert sind.

Da bei diesem Verfahren eine Vervielfältigung der Zielsequenzen nicht nötig ist, sollten in der Probe hinreichend viele Kopien der Zielsequenz vorliegen. Dies ist z.B. zu erwarten im Falle von mRNA, rRNA sowie bei Proben, die sehr viele Zellen enthalten. Selbstverständlich ist das erfindungsgemäße Verfahren auch bei Proben anwendbar, die vorher, z.B. mit PCR oder SDA, amplifiziert worden sind.

Im Rahmen der Erfindung kann eine Probe insbesondere eine biologische oder klinische Probe sein, z.B. ein Nukleinsäuren umfassender Extrakt aus Zellen pflanzlichen, tierischen oder humanen Ursprungs. Die Erfindung kann z.B. vorteilhaft zur Detektion von Nukleinsäuren eines Pathogens in einer klinischen Probe oder zur Detektion von gentechnisch veränderter DNA bekannter Sequenz in einer pflanzlichen oder Nahrungsmittel-Probe eingesetzt werden.

Der wirtschaftliche Nutzen der Erfindung zeigt sich z.B. darin, dass die sequenzspezifische Analytik von DNA und RNA erheblich vereinfacht wird, weil keine PCR mehr erforderlich ist. Es können elektrochemische DNA-Chips zum Einsatz kommen, welche direkt auslesbare digitale Daten liefern. Es können durch Verwendung von Elektrodenarrays sehr viele unterschiedliche Sequenzen parallel detektiert werden. Es sind keine teuren, speziell fluoreszenz-markierten Oligonukleotide mehr nötig. Auch im Falle der Genexpressionsanalysen kann die teure und langwierige PCR-Vervielfältigung entfallen. Außerdem ist eine direkte Quantifizierung gegeben, weil durch Wegfall der PCR die elektrochemischen Signale direkt proportional zur Konzentration der Zielsequenzen sind.

Insbesondere eröffnen sich folgende wirtschaftlich interessante Anwendungen des erfindungsgemäßen Prinzips:
1. Direkte quantitative Detektion einer gesuchten DNA-Sequenz bei großem Überschuss an unspezifischer DNA ohne PCR, beispielsweise zur quantitativen Analyse gentechnisch veränderter Organismen.
2. Schnelle, einfache, kostengünstige elektrochemische Analytik von mRNA-Strängen aus der Genexpression, quantitativ und ohne vorherige PCR.
3. Quantitative, schnelle, einfache, kostengünstige elektrochemische Analytik von Bakterien über deren spezifische rRNA in klinischen Proben,
4. Schnelle, einfache, kostengünstige elektrochemische Multiplex-Detektierung vieler Nukleinsäure-Sequenzen in einer Probe mittels Elektrodenarrays, ohne dass eine kompromissbehaftete Multiplex-PCR nötig wäre,
5. Selektive und sensitive Detektion von PCR-Produkten.

### Legende

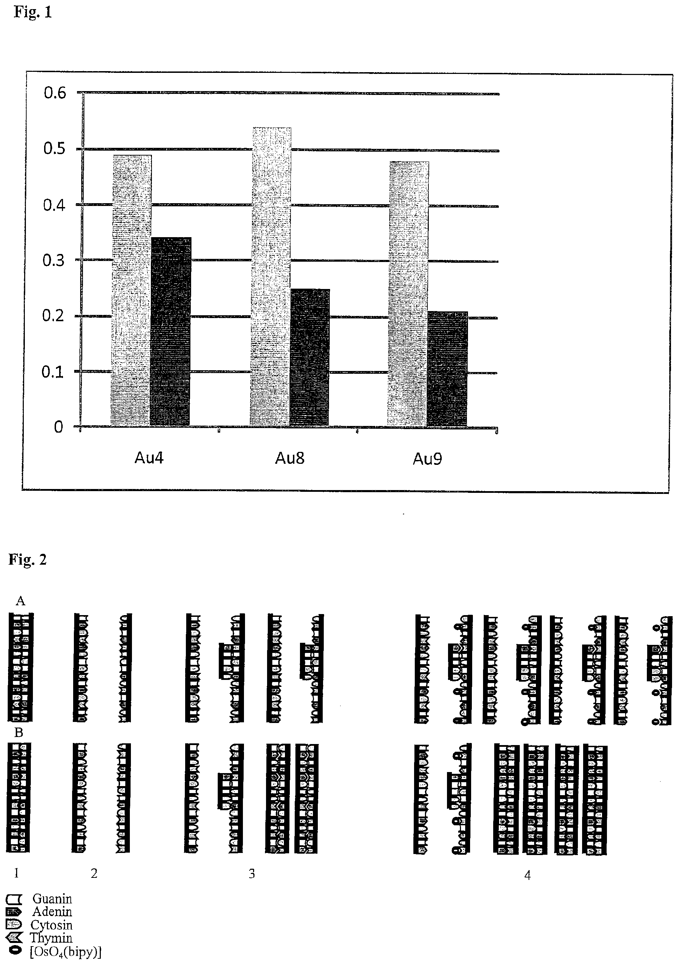

- **Fig. 1**: zeigt die Ergebnisse der Markierung von PCR-Produkten mit [OsO₄(bipy)] bei unterschiedlichen Methoden der Hybridisierung mit Schutzstrang (siehe Beispiel 1). Linker Balken: Verfahren 1, rechter Balken: Verfahren 2. SWV-Signale in Mikroampere.
- **Fig. 2**: (A) zeigt die Methode zur Markierung von PCR-Produkten nach Verfahren 1, (B) zeigt die Methode nach Verfahren 2; 1. zeigt die doppelsträngigen PCR-Produkte; 2. stellt die Stränge nach der Denaturierung dar; 3. zeigt die Situation nach dem Abschrecken, in Verfahren 1 bleiben die Einzelstränge länger von ihren komplementären Strängen getrennt, so dass die Schutzstränge effektiver hybridisieren können; 4. zeigt die Situation nach der Markierung mit (OsO₄[bipy]), es liegen bei Verfahren 1 deutlich mehr markierte Targets vor, die mit den Sonden an der Elektrode hybridisieren können

### Beispiele

### Beispiel 1: Markierung von doppelsträngigen PCR-Produkten mit [OsO₄(bipy)]

Für die Markierung von PCR-Produkten mit [OsO₄(bipy)] wurden aus einer klinischen Probe, welche *Candida albicans*-DNA enthielt, mehrere identische PCR-Ansätze hergestellt und eine PCR durchgeführt (*Candida albicans:* Primer: S1: 5'-ACTGCGAATGGCTCATTAAATCAG-3' (SEQ ID NO:1), CUF1: 5'-CAAGGCCATGCGATTCG-3' (SEQ ID NO:2); Sonde: V2CA: 5'-TGCCTTCGGGCTCTTTGA-aaaaaaaaaaaaaaa[Dithio]₃-3' (SEQ ID NO:3); Schutzstrang: Schutz Canda: 5'- TACCTTCAGGCTCTTTAG-3' (SEQ ID NO:4).

Die Hybridisierung mit Schutzsträngen wurde unterschiedlich durchgeführt, nach den im Folgenden beschriebenen Verfahren 1 (erfindungsgemäß) und 2 (erfindungsgemäß modifizierte Methode von Th. Reske):
1. Erfindungsgemäß wurde die Probe wurde im Thermocycler bei 95 °C für 10 min denaturiert. Das Gefäß mit Reaktionsmischung wurde dann in eine Kältemischung (Eisbad mit Salz (NaCl)) transferiert und die Reaktionsmischung dadurch sehr schnell auf -2,5 °C abgekühlt. Der Schutzstrang wurde zugegeben und für 10 min bei dieser Temperatur inkubiert. Dann wurde für weitere maximal 110 min bei Raumtemperatur inkubiert.
2. Nach Stand der Technik (Reske, Dissertation 2009, S. 18) wurde die Probe nur kurz bei 95 °C für max. 1 min denaturiert. Diese Versuche waren nicht erfolgreich, d.h. es wurden nur sehr kleine Signale erhalten. Erst durch erfindungsgemäße längere Haltezeiten für Denaturierung und Schutzstrang-Hybridisierung konnten brauchbare, aber relativ kleine Signale am Ende erhalten werden. In diesem Beispiel wurde daher schon länger als im Stand der Technik, d.h. erfindungsgemäß für 10 min denaturiert. Danach wurde sie in ein Eisbad (0-2 °C) überführt und dort der Schutzstrang zugegeben.

Die Reaktionsmischung wurde nun erfindungsgemäß längere Zeit, d.h. für 10 min in dem Eisbad gekühlt, dann für weitere maximal 110 min bei Raumtemperatur inkubiert.

Danach erfolgten bei allen Proben die Zugabe von [OsO₄(bipy)] zu einer Endkonzentration von 2 mM und eine weitere Inkubation von 2 h.

Die Entfernung von [OsO₄(bipy)] erfolgte in beiden Fällen über Nacht durch Dialyse.

Ergebnisse sind in Fig. 1 gezeigt. Die nach der erfindungsgemäß modifizierten Methode von Thomas Reske hergestellten Targets (Verfahren 2) zeigen deutlich niedrigere Signale an drei unabhängig voneinander präparierten Elektroden (Au4, Au8, Au9).

Die Hybridisierung der so markierten Targets an die Sonden wurde bei Raumtemperatur für 15 min durchgeführt. Die verwendete Messmethode war die SWV (Square Wave Voltammetry): Scan -0.55 V - 0V, 200 Hz, Step Potential 0.002 V, Amplitude 0.04 V, verwendeter Puffer: 10 mM Tris, 0.5 M Na₂SO₄, pH 7.5.

### Beispiel 2: Detektion von 0,9%-Spuren des MON810-Gens in der DNA einer Maisprobe durch Markierung mit [OsO₄(bipy)], Hybridisierung und elektrochemische Analyse.

Es wird eine PCR-Reaktion mit einem Template durchgeführt, das 0,9 % [w/w] an transgener DNA (MON810-Gen) enthält. Bei der PCR wird ein transgener Abschnitt amplifiziert. Anschließend wird zur Denaturierung aller Doppelstränge auf 95 °C für 10 min aufgeheizt. Dann wird der Schutzstrang im Überschuss zugesetzt. Dann wird die Probe erfindungsgemäß in einer Kältemischung aus Eis/NaCl für 10 min auf -2,5 °C abgekühlt. Nun wird der Schutzstrang thermisch stringent an die passenden Sequenzen in der Mais-DNA angelagert. Anschließend wird [OsO₄(bipy)] (bevorzugt 2 mM) zugesetzt. Nach Ablauf der Reaktion kann das überschüssige [OsO₄(bipy)] durch Dialyse abgetrennt werden. Zum Schluss erfolgt die Hybridisierung mit an Gold immobilisierten Sondensträngen unter Verdrängung der Schutzstränge und die elektrochemische Detektierung z.B. mittels SWV. Der große Überschuss an unspezifischer DNA und RNA wurde im Markierungsschritt mit dem Osmiumtetroxid-Komplex markiert und ist seitdem nicht mehr zur Hybridisierung fähig. Daher erhält man nur dann voltammetrische Signale des Osmiumtetroxid-Komplex-Markers, wenn der Schutzstrang sein Gegenstück (MON810-Abschnitt) in der amplifizierten Mais-DNA finden und damit hybridisieren konnte.

### Beispiel 3: Untersuchung der Genexpression durch Markierung der mRNA-Kopien mit [OsO₄(bipy)].

Die Nukleinsäureextrakte einer Zell- oder Gewebeprobe werden bei 95 Grad denaturiert und am Ende der Denaturierung mit den Schutzsträngen versetzt, mittels Kältemischung auf -2,5 °C abgeschreckt und anschließend bei Raumtemperatur mit den Schutzsträngen hybridisiert. Das Markierungsprinzip folgt dabei dem in Beispiel 1 vorgestellten. Allerdings ist hier darauf zu achten, dass bis zur Markierung der RNA auf RNase-Freiheit geachtet wird. Dann wird [OsO₄(bipy)] (bevorzugt 2 mM) zugesetzt. Der größte Teil der Nukleinsäurestränge (DNA und RNA) liegt dabei einzelsträngig vor und wird dabei mit Osmiumtetroxid modifiziert und ist nicht mehr zur Bildung von Doppelsträngen in der Lage. Nur die geschützten Abschnitte der mRNA-Kopien bleiben unmarkiert und können daher anschließend mit den an Gold immobilisierten Sondensträngen unter Verdrängung der Schutzstränge hybridisieren. Dadurch werden einerseits eine hohe Selektivität und andererseits eine sehr hohe Empfindlichkeit bei der anschließenden elektrochemischen Analyse beispielsweise durch Chronopotentiometrie erreicht. Eine Vervielfältigung der mRNA-Kopien durch RT-PCR und PCR kann entfallen.

### Beispiel 4: Detektion von Bakterienzellen durch Markierun der spezifischen rRNA mit [OsO₄(bipy)].

Die Nukleinsäureextrakte einer klinischen Probe werden bei 95 Grad denaturiert und mit den Schutzsträngen versetzt, erfindungsgemäß sehr schnell mittels Kältemischung auf - 2,5 °C abgeschreckt und anschließend bei Raumtemperatur mit den Schutzsträngen hybridisiert. Dann wird [OsO₄(bipy)] (bevorzugt 2 mM) zugesetzt. Der größte Teil der Nukleinsäurestränge (DNA und RNA) liegt dabei einzelsträngig vor, wird mit Osmiumtetroxid-Bipyridin modifiziert und ist nicht mehr zur Bildung von Doppelsträngen in der Lage. Nur die geschützten Abschnitte der rRNA-Kopien können anschließend mit den an Gold immobilisierten Sondensträngen unter Verdrängung der Schutzstränge hybridisieren. Dadurch werden einerseits eine hohe Selektivität und andererseits eine sehr hohe Empfindlichkeit bei der anschließenden elektrochemischen Analyse beispielsweise durch Chronopotentiometrie erreicht. Eine Vervielfältigung der rRNA-Kopien durch RT-PCR und PCR entfällt komplett. Dieses Prinzip kann zur Identifizierung und Quantifizierung infektiöser Bakterien in klinischen Proben verwendet werden. Auch in diesem Fall ist es wesentlich, auf RNase-Freiheit zu achten, bis die RNA mit [OsO₄(bipy)] markiert ist.

### Beispiel 5: Elektrochemische Analyse der Os-markierten Nukleinsäuren direkt im Anschluss an die Markierungsreaktion ohne vorherige Entfernung des überschüssigen gelösten [OsO₄(bipy)].

Durch Anwendung der Chronocoulometrie kann man zwischen den elektrochemischen Signalen der immobilisierten Osmiumverbindungen und denen der gelösten [OsO₄(bipy)]-Moleküle unterscheiden. Daher ist eine Entfernung überschüssiger Osmiumkomplexe nicht notwendig. Zur Durchführung müssen die immobilisierten Sonden mit passenden SondenSchutzsträngen geschützt sein. Letztere sind komplementär zu den immobilisierten Sonden, enthalten vorzugsweise aber ebenfalls 3 bis 5, bevorzugt 4 Basenfehlpaarungen, um die Hybridisierung von Sonde und Target zu fördern. Der Potentialsprung erfolgt von -500 zu -200 mV (vs. SCE). Zur Auswertung wird analog zum Verfahren nach Steel et al. (Steel et al., Anal. Chem. 1998, 70, 4670-4677, Fig. 4) verfahren, d.h. die Auftragung der chronocoulometrischen Messwerte erfolgt gegen die Quadratwurzel der Zeit. Der lineare Abschnitt wird bis t = 0 extrapoliert, der Schnittpunkt dieser Geraden mit der Ladungsachse ergibt die Ladung als Messwert. Hiervon ist noch der Leerwert abzuziehen, der durch die kapazitive Umladung der elektrochemischen Doppelschicht entsteht. Die resultierende Ladung ist auf den elektrochemischen Umsatz der an der Sondenschicht mit dem Target immobilisierten Marker zurückzuführen und proportional zur Targetkonzentration.

### Beispiel 6: Elektrochemische Analyse der Os-markierten Nukleinsäuresequenzen durch thermisch stringente Hybridisierung an selektiv geheizten Elektrodenarrays.

Jede einzelne Elektrode wird bei der Analyse auf eine Temperatur gebracht, bei der die darauf immobilisierte Fängersonde optimal und thermisch stringent mit der Zielsequenz hybridisiert. Dies unterstützt zusätzlich die spezifische Detektion der unterschiedlichen Zielsequenzen in Gegenwart eines großen Überschusses an unspezifischen Nukleinsäuren. Elektrodenarrays, welche im Rahmen der Erfindung verwendet werden können, sind z.B. in der Dissertation von Duwensee H., 2009 oder in DE 10 2004 017 750 B4 beschrieben.

### Beispiel 7: Elektrochemische Analyse der Os-markierten Sequenzen an nanostrukturierten Elektroden.

Die Abscheidung von Nanostrukturen aus Gold und/oder Silber vergrößert die aktive Elektroden-Oberfläche und verbessert sowohl die Hybridisierungseffizienz und Stringenz, als auch die Empfindlichkeit und das Signal-Rausch-Verhältnis. Daher werden die Nachweisgrenze sowie die Selektivität weiter verbessert. Geeignete Nanostrukturen sind bei Wachholz et al., Electroanalysis 21 (19) (2009) 2153 oder French R et al. J Electroanal Chem 632 (2009) 206 und French R et al. Electroanalysis 20 (22) (2008) 2403 beschrieben. Die Abscheidung kann sowohl aus cyanidischen als auch aus anderen galvanischen Bädern nach dem Stand der Technik erfolgen.

### Literatur;

Foita et al., 1 Am. Chem. Soc. 126 (2004) 6532.
Kostecka et al., Bioelectrochemistry 63 (2004) 245.
Foita et al., Electroanalysis 15 (2003) 431.
Flechsig et al., Langmuir 21 (2005) 7848.
Flechsig et al., Anal. Chem 79 (2007) 2125.
French R et al., J Electroanal Chem 632 (2009) 206
French R et al., Electroanalysis 20 (22) (2008) 2403
Reske et al., Talanta 74 (2007) 393
Mix et al., Electroanalysis 21 (2009) 826
F. Wachholz et al., Electroanalysis 21 (2009) 2153
Steel et al., Anal. Chem. 1998, 70, 4670-4677
H. Duwensee, M. Mix, G.-U. Flechsig, Bioanalytical Reviews 1-4 (2010) 103
Reske, Dissertation, 2009
DE 10 2005 039 726
DE 10 2004 017 750 B4
WO 07/020093

## Patentansprüche

1. Verfahren zum sequenzspezifischen Nachweis von Nukleinsäuren in einer Probe, in der die Nukleinsäuren zumindest partiell als Doppelstrang vorliegen, Schritte umfassend, bei denen man
a) die zumindest partiell doppelsträngigen Nukleinsäurestränge durch thermische Denaturierung in Einzelstränge überführt, welche als Targetstränge bezeichnet werden, wobei die thermische Denaturierung bei mindestens 93°C für mindestens 10 min stattfindet,
b) mindestens einen als Schutzstrang bezeichneten Nukleinsäurestrang hinzufügt, welcher mit einem Targetstrang hybridisieren kann, um partielle Doppelstrangabschnitte auszubilden, wobei die Schutzstränge kürzer als die Targetstränge sind, wobei die Temperatur der Probe schnell auf eine Temperatur von weniger als 0°C gesenkt wird, indem die Probe sofort nach Hinzufügen des Schutzstrangs in Schritt b) in eine Umgebung von weniger als 0°C überführt wird, wobei die Temperatur mindestens 10 min gehalten wird,
c) die verbliebenen Einzelstrangabschnitte der Targetstränge durch Reaktion mit einem Redoxmarker markiert, der selektiv mit der Doppelbindung der Pyrimidinringe der Nukleinsäurestränge reagieren und eine elektroanalytisch nutzbare Redoxreaktion an Arbeitselektroden ermöglicht, wobei der Redoxmarker [OsO₄(bipy)] oder [Os0₄(py)₂] ist, man
d) die so markierten Nukleinsäurestränge an der Oberfläche einer Elektrode mit dort immobilisierten Sondensträngen unter Verdrängung der Schutzstränge hybridisiert und man
e) die an den Sondensträngen hybridisierten Nukleinsäurestränge elektroanalytisch nachweist.

2. Verfahren nach Anspruch 1, **dadurch gekennzeichnet, dass** die thermische Denaturierung in Schritt a) bei 95°C für mindestens 10 min stattfindet.

3. Verfahren nach einem der Ansprüche 1 oder 2, **dadurch gekennzeichnet, dass** das Hinzufügen des Schutzstranges in Schritt b) etwa bei der Temperatur der thermischen Denaturierung erfolgt.

4. Verfahren nach einem der vorherigen Ansprüche, **dadurch gekennzeichnet, dass** die Temperatur der Probe in Schritt b) ca. -1,5°C bis-3 °C beträgt, bevorzugt -2,5°C wobei diese Temperatur insbesondere in einer Kältemischung aus Eis mit einem Salz, bevorzugt NaCl, erreicht wird.

5. Verfahren nach einem der vorherigen Ansprüche, wobei der Schutzstrang höchstens halb so lang ist wie der Targetstrang, wobei der Schutzstrang bevorzugt eine Länge von 10-200 b aufweist, mehr bevorzugt von 20-30 b, wobei der Schutzstrang bevorzugt so ausgestaltet ist, dass in den Doppelstrangabschnitten aus Schutzstrang und Targetstrang eine oder mehrere Fehlpaarungen auftreten, und/oder wobei der Schutzstrang in einem Überschuss von mindestens 2:1, bevorzugt 3:1, 4:1, 5:1, 9:1 oder 10:1 hinzugefügt wird.

6. Verfahren nach einem der vorherigen Ansprüche, **dadurch gekennzeichnet, dass** die Nukleinsäuren in der Probe RNA und/oder DNA sind.

7. Verfahren nach einem der vorherigen Ansprüche, **dadurch gekennzeichnet, dass** die Nukleinsäuren in der Probe vor der Denaturierung in Schritt a) in kürzere Abschnitte geteilt werden, z.B. von jeweils 100 bis 5000 Basenpaaren, vorzugsweise 500 bis 2000 Basenpaaren, wobei dies optional mit Nuklease-Behandlung oder Restriktionsendonuklease-Behandlung erreicht werden kann.

8. Verfahren nach einem der vorherigen Ansprüche, **dadurch gekennzeichnet, dass** die Verdrängung der Schutzstränge durch die immobilisierten Sondenstränge bei einer Temperatur erfolgt, die optimal für die thermisch stringente Hybridisierung von Sonden und Targetsträngen ist, wobei die Sondenstränge bevorzugt an einer beheizten Elektrode immobilisiert sind, wobei optional mehrere unterschiedliche Sonden verwendet werden, die an unterschiedlichen selektiv beheizten Elektroden immobilisiert sind, um während der Hybridisierung und/oder Messung die für jede Sondensequenz jeweils optimale Temperatur einzustellen.

9. Verfahren nach einem der vorherigen Ansprüche, **dadurch gekennzeichnet, dass** zwischen Schritt c) und d) die überschüssigen Redoxmarker aus der Analysenlösung entfernt werden, z.B. durch Dialyse oder Filtration.

10. Verfahren nach einem der vorherigen Ansprüche, **dadurch gekennzeichnet, dass** zwischen Schritt c) und d) die überschüssigen Redoxmarker aus der Analysenlösung nicht entfernt werden, sondern in der Probe verbleiben, wobei die elektrochemischen Signale des oberflächlich gebundenen Osmiums von den Signalen des in Lösung befindlichen Osmiums bei der Detektierung durch geeignete elektrochemische Analyseverfahren, vorzugsweise durch Chronocoulometrie oder Chronopotentiometrie, voneinander getrennt werden.

11. Verfahren nach einem der vorherigen Ansprüche, **dadurch gekennzeichnet, dass** es in einer Vorrichtung durchgeführt wird, umfassend ein Fließsystem, in welchem die Schritte des Verfahrens nacheinander stattfinden, wobei es in dem Fließsystem heizbare Abschnitte zur thermischen Denaturierung sowie kühlbare Abschnitte zur schnellen Kühlung der Probe gibt, wobei der Abschnitt zur thermischen Denaturierung auf 93-97°C beheizbar ist, wobei das System so ausgestaltet ist, dass nach der thermischen Denaturierung Schutzstrang hinzugefügt werden kann, wobei die Probe direkt danach in einen Abschnitt geleitet werden kann, der auf eine Temperatur von -2,5°C bis -25°C kühlbar ist.

12. Verfahren nach Anspruch 11, **dadurch gekennzeichnet, dass** die Vorrichtung als elektrochemischen Detektor zum elektroanalytischen Nachweis der an den Sondensträngen hybridisierten Nukleinsäurestränge ein Array aus unterschiedlichen selektiv heizbaren Arbeitselektroden umfasst, wobei an den Arbeitselektroden unterschiedliche Sonden immobilisiert sind.

## Claims

1. A method for the sequence-specific detection of nucleic acids in a sample, in which the nucleic acids are at least partially present as double strands, comprising
a) converting the at least partially double stranded nucleic acid strands into single strands termed target strands by thermal denaturation, wherein the thermal denaturation takes place at at least 93° C for at least 10 min,
b) adding at least one nucleic acid strand termed protective strand, which is able to hybridize with a target strand to form partially double stranded segments, wherein the protective strands are shorter than the target strands, wherein the temperature of the sample is rapidly lowered to a temperature of less than 0°C by transferring the sample to an environment of less than 0° C immediately after addition of the protective strand in step b), wherein the temperature is maintained for at least 10 min,
c) labeling the remaining single stranded segments of the target strands via reaction with a redox marker, which reacts selectively with the double bond of the pyrimidine rings of the nucleic acid strands and allows an electroanalytically usable redox reaction on working electrodes, wherein the redox marker is [OsO₄(bipy)] or [OsO₄(py)₂],
d) hybridizing the nucleic acid strands that are labeled in this manner on the surface of an electrode with probe strands that are immobilized thereon under replacement of the protective strands, and
e) electroanalytically detecting the nucleic acid strands that are hybridized to the probe strands.

2. The method according to claim 1, wherein the thermal denaturation in step a) takes place at 95° C for at least 10 min.

3. The method according to any of claims 1 or 2, wherein the addition of the protective strand in step b) takes place at a temperature of approximately the thermal denaturation temperature.

4. The method according to any of the preceding claims, wherein the temperature of the sample in step b) is about -1.5° C to -3° C, wherein this temperature is preferably achieved in a freezing mixture of ice with a salt, preferably, NaCl.

5. The method according to any of the preceding claims,
wherein the protective strand is half as long as the target strand at most,
wherein the protective strand preferably has a length of 10-200 b, more preferably,
of 20-30 b,
wherein the protective strand is preferably designed in such a manner that one or more mismatches occur in the double stranded segments of protective strand and target strand, and/or wherein the protective strand is added in an excess of at least 2:1, preferably, 3:1, 4:1,5:1,9:1 or 10:1.

6. The method according to any of the preceding claims, wherein the nucleic acids in the sample are RNA and/or DNA.

7. The method according to any of the preceding claims, wherein the nucleic acids in the sample are separated into shorter segments before the denaturation in step a), e.g., of 100 to 5000 base pairs each, preferably, 500 to 2000 base pairs,
wherein this can optionally be achieved by treatment with nuclease or restriction endonuclease.

8. The method according to any of the preceding claims, wherein the replacement of the protective strands by the immobilized probe strands occurs at a temperature that is optimal for the thermally stringent hybridization of probe and target strands,
wherein the probe strands are preferably immobilized on a heatable electrode,
wherein, optionally, several different probes immobilized on different selectively heated electrodes are used to obtain the respective optimal temperature for each probe sequence during hybridization and/or detection.

9. The method according to any of the preceding claims, wherein the surplus redox markers are removed from the analyte solution between step c) and d), e.g., by means of dialysis or filtration.

10. The method according to any of the preceding claims, wherein the surplus redox markers are not removed from the analyte solution between step c) and d), but remain in the sample, wherein the electrochemical signals of the osmium bound on a surface are separated from the signals of the osmium in solution during the detection by means of suitable electrochemical analysis methods, preferably, by chronocoulometry or chronopotentiometry.

11. The method according to any of the preceding claims, which is carried out in a device comprising a flow system in which the method steps take place in succession, wherein the flow system has heatable sections for thermal denaturation as well as coolable sections for rapid cooling of the sample, wherein the section for thermal denaturation is heatable to 93-97°C, wherein the system is designed in a manner which allows for addition of protective strand after thermal denaturation, wherein the sample can directly afterwards be directed to a section coolable to a temperature of -2.5°C to -25°C.

12. The method according to claim 11, wherein the device comprises an array of different, selectively heatable working electrodes as an electrochemical detector for electroanalytical detection of the nucleic acid strands hybridized to the probe strands, wherein different probes are immobilized on the working electrodes.

## Revendications

1. Procédé de détection, à spécificité de séquence, d'acides nucléiques au sein d'un échantillon, dans lequel les acides nucléiques se présentent, au moins partiellement, sous la forme double-brin, lequel procédé comporte les étapes suivantes :
a) convertir les brins d'acides nucléiques se présentant, au moins partiellement, sous la forme double-brin en simples brins appelés brins cibles, par dénaturation thermique, laquelle dénaturation thermique est effectuée à une température d'au moins 93 °C pendant un laps de temps d'au moins 10 minutes ;
b) ajouter au moins un brin d'acide nucléique, appelé brin protecteur, qui est capable de s'hybrider avec un brin cible pour former des segments partiellement double-brins, lesquels brins protecteurs sont plus courts que les brins cibles, puis abaisser rapidement la température de l'échantillon à moins de 0 °C en transférant l'échantillon dans un environnement où règne une température inférieure à 0 °C, immédiatement après l'addition du ou des brin(s) protecteur(s) dans l'étape (b), et maintenir l'échantillon à cette température pendant au moins 10 minutes ;
c) marquer les segments simple-brins restants des brins cibles, par réaction avec un marqueur redox qui réagit sélectivement avec la double liaison des cycles pyrimidiniques des brins d'acide nucléique et permet une réaction redox, utilisable en analyse électrochimique, sur des électrodes de travail, lequel marqueur redox est [OsO₄(bipy)] ou [OsO₄(py)₂] ;
d) hybrider les brins d'acide nucléiques ainsi marqués, sur la surface d'une électrode, avec des brins sondes qui y sont immobilisés, en remplacement des brins protecteurs ;
e) et détecter, par analyse électrochimique, les brins d'acide nucléique qui sont hybridés aux brins sondes.

2. Procédé conforme à la revendication 1, dans lequel, dans l'étape (a), la dénaturation thermique est réalisée à 95 °C durant au moins 10 minutes.

3. Procédé conforme à la revendication 1 ou 2, dans lequel, dans l'étape (b), l'addition du brin protecteur est réalisée à une température voisine de la température de dénaturation thermique.

4. Procédé conforme à l'une des revendications précédentes, dans lequel, dans l'étape (b), la température de l'échantillon vaut à peu près de -1,5 à -3 °C, laquelle température est de préférence atteinte au moyen d'un mélange réfrigérant de glace et d'un sel, de préférence NaCl.

5. Procédé conforme à l'une des revendications précédentes, dans lequel
- le brin protecteur est au plus moitié aussi long que le brin cible,
- le brin protecteur est long de préférence de 10 à 200 bases, et mieux encore de 20 à 30 bases,
- le brin protecteur est de préférence conçu de telle sorte qu'il y ait un ou plusieurs mésappariements dans les segments double-brins formés d'un brin protecteur et d'un brin cible,
- et/ou le brin protecteur est ajouté en excès, en un rapport d'au moins 2/1, et de préférence 3/1, 4/1, 5/1, 9/1 ou 10/1.

6. Procédé conforme à l'une des revendications précédentes, dans lequel les acides nucléiques présents dans l'échantillon sont des ARN et/ou des ADN.

7. Procédé conforme à l'une des revendications précédentes, dans lequel, avant d'être dénaturés dans l'étape (c), les acides nucléiques présents dans l'échantillon sont séparés en segments plus courts, par exemple de 100 à 5000 paires de bases chacun, et de préférence, de 500 à 2000 paires de bases,
étant entendu que, en option, ceci peut être réalisé par traitement avec une nucléase ou une endonucléase de restriction.

8. Procédé conforme à l'une des revendications précédentes, dans lequel le remplacement des brins protecteurs par les brins sondes immobilisés est opéré à une température optimale pour l'hybridation, dans des conditions thermiques stringentes, des brins sondes et des brins cibles,
étant entendu que les brins cibles sont de préférence immobilisés sur une électrode qui peut être chauffée,
et étant entendu que, en option, on utilise plusieurs sondes différentes, immobilisées sur des électrodes différentes chauffées sélectivement, afin d'atteindre, pendant l'hybridation et/ou la détection, la température optimale respective pour chaque séquence sonde.

9. Procédé conforme à l'une des revendications précédentes, dans lequel, entre les étapes (c) et (d), on élimine l'excès de marqueur redox de la solution d'analyte, par exemple par dialyse ou filtration.

10. Procédé conforme à l'une des revendications précédentes, dans lequel on n'élimine pas l'excès de marqueur redox de la solution d'analyte entre les étapes (c) et (d), mais celui-ci reste dans l'échantillon et les signaux électrochimiques de l'osmium fixé sur une surface sont, au cours de la détection, séparés des signaux de l'osmium en solution au moyen de méthodes appropriées d'analyse électrochimique, et de préférence, par chronocoulométrie ou chronopotentiométrie.

11. Procédé conforme à l'une des revendications précédentes, qui est mis en oeuvre dans un dispositif comprenant un système opérant en mode continu dans lequel se déroulent successivement les étapes du procédé, étant entendu que ce système continu comporte des sections chauffables pour la dénaturation thermique ainsi que des sections réfrigérables pour le refroidissement rapide de l'échantillon, que la section de dénaturation thermique peut être chauffée à une température de 93 à 97 °C, que le système est conçu de manière à permettre l'addition de brins protecteurs après la dénaturation thermique, et que l'échantillon peut être ensuite directement dirigé vers une section qui peut être réfrigérée à une température de -2,5 à -25 °C.

12. Procédé conforme à la revendication 11, dans lequel le dispositif comprend un arrangement de différentes électrodes de travail sélectivement chauffables, qui sert de détecteur électrochimique pour la détection, par analyse électrochimique, des brins d'acide nucléique hybridés aux brins sondes et où des sondes différentes sont immobilisées sur les électrodes de travail.


### SEQUENCE LISTING

<110> Universität Rostock
<120> Sequenzspezifische Analyse von Nukleinsauren
<130> P88987
<160> 4
<170> PatentIn version 3.5
<210> 1
   <211> 24
   <212> DNA
   <213> Artificial Sequence
<220>
   <223> Primer S1
<400> 1
   actgcgaatg gctcattaaa tcag 24
<210> 2
   <211> 17
   <212> DNA
   <213> Artificial Sequence
<220>
   <223> Primer CUF1
<400> 2
   caaggccatg cgattcg 17
<210> 3
   <211> 33
   <212> DNA
   <213> Artificial Sequence
<220>
   <223> Sonde V2CA
<400> 3
   tgccttcggg ctctttgaaa aaaaaaaaaa aaa 33
<210> 4
   <211> 18
   <212> DNA
   <213> Artificial Sequence
<220>
   <223> Schutz Canda
<400> 4
   taccttcagg ctctttag 18